# EUROPEAN PATENT SPECIFICATION

(11) **EP 3 290 022 B1**
(45) Date of publication and mention of the grant of the patent: **01.04.2020**
(21) Application number: 16186853.4
(22) Date of filing: 01.09.2016
(51) Int. Cl.: A61K 9/00, A61K 31/366, A61P 9/10

(54) **USE OF STATINS IN THE TREATMENT OF ISCHEMIC DISEASES**
VERWENDUNG VON STATINEN IN DER BEHANDLUNG VON ISCHÄMISCHEN KRANKHEITEN
UTILISATION DE STATINES DANS LE TRAITEMENT DE MALADIES ISCHÉMIQUES

(43) Date of publication of application: 07.03.2018
(73) Proprietor: Peking University Third Hospital, Haidian District, Beijing 100191 (CN)
(72) Inventor: SONG, Chunli, Haidian District, Beijing 100191 (CN); XU, Yingsheng, Haidian District, Beijing 100191 (CN); TAN, Jie, Haidian District, Beijing 100191 (CN); GUO, Qi, Haidian District, Beijing 100191 (CN); LIU, Can, Haidian District, Beijing 100191 (CN)
(74) Representative: Gregson, Anna Louise

(56) References cited:
- US-A1- 2004 122 077
- US-A1- 2014 323 536
- Hazem F Elewa ET AL: "Diverse Effects of Statins on Angiogenesis: New Therapeutic Avenues", Pharmacotherapy, 1 February 2010 (2010-02-01), pages 169-176, XP055323996, Retrieved from the Internet: URL:http://onlinelibrary.wiley.com/store/1 0.1592/phco.30.2.169/asset/phco.30.2.169.p df?v=1&t=iw3cnrva&s=50c5eb20eebce31d9cf92e ba1ce6344275e11928 [retrieved on 2016-11-29]
- YUQING ZHANG ET AL: "Simvastatin augments the efficacy of therapeutic angiogenesis induced by bone marrow-derived mesenchymal stem cells in a murine model of hindlimb ischemia", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 1, 7 May 2011 (2011-05-07), pages 285-293, XP019981519, ISSN: 1573-4978, DOI: 10.1007/S11033-011-0737-Y
- TAN J ET AL: "A single CT-guided percutaneous intraosseous injection of thermosensitive simvastatin/poloxamer 407 hydrogel enhances vertebral bone formation in ovariectomized minipigs", OSTEOPOROSIS INTERNATIONAL, SPRINGER, GB, vol. 27, no. 2, 30 July 2015 (2015-07-30), pages 757-767, XP035866159, ISSN: 0937-941X, DOI: 10.1007/S00198-015-3230-Y [retrieved on 2015-07-30]

## Description

### Field of the Invention

The present invention relates to the field of pharmaceutical biotechnology, more specifically, the new use of HMG-CoA reductase inhibitors (statins) for the treatment of ischemic diseases.

### Background of the Invention

Cardio-cerebrovascular diseases cause great harm to human health, and the resulting morbidity and disability is very high. With the aging population and the decline in the age of onset, the problem is more and more serious. At present, the treatment methods of ischemic heart/cerebral blood vessels mainly include drugs, angioplasty and artery bypass grafting. Current randomized control trial (RCT) research shows that positive drug treatment can achieve the same or even better results than the interventional angioplasty treatment (Lampropoulos CE, Papaioannou I, D'Cruz DP. Osteoporosis--a risk factor for cardiovascular disease? Nature reviews Rheumatology. 2012;8:587-98. This has led us reflection on the interventional therapy and the exploration of the treatment of revascularization.

Peripheral arterial disease (PAD) is caused by occlusive atherosclerosis in a vascular bed other than the heart. PAD is now known to be an important public health problem with a total prevalence that is nearly equal to that of coronary artery disease (CAD). The prevalence of PAD increases with age, affecting 6% of individuals aged 50-60 years, and 10-20% of individuals aged >70 years. Despite improvements in medical care and revascularization, patients with critical limb ischaemia continue to have a high risk of major amputation (below the knee or higher) and cardiovascular death. The primary goal of therapy in critical limb ischaemia is to achieve blood flow to the distal limb vessels with angioplasty or bypass surgery. However, many patients with critical limb ischaemia are unsuitable for revascularization, and the long-term re-occlusion rate is high, and the outflow tract of patients with extensive occlusion cannot be operated at all.

Bone marrow derived endothelial progenitor cells (Endothelial Progenitor Cells, EPCs) play important roles in angiogenesis and endothelial homeostasis. Therapeutic angiogenesis, also known as "drug bypass", by using exogenous vascular growth factor and bone marrow derived EPCs, to promote angiogenesis in ischemic tissue (Therapeutic angiogenesis for critical limb ischaemia. Nature Reviews Cardiology. 2013; 10:387-396.). However, the limitation of therapeutic angiogenesis is the number of circulating EPCs is low, aging, diabetes, hyperlipidaemia and other diseased cause the number of circulating EPCs even less (Yao L, et al. Bone marrow endothelial progenitors augment atherosclerotic plaque regression in a mouse model of plasma lipid lowering. Stem Cells. 2012;30:2720-2731.) (Adler BJ, et al. Obesity-driven disruption of haematopoiesis and the bone marrow niche. Nature Reviews Endocrinology. 2014;10:737-748.) . It is an important therapeutic strategy to increase the number of peripheral blood EPCs by promoting endogenous EPCs mobilization (Liu Y, et al. Beneficial effects of statins on endothelial progenitor cells. Am J Med Sci. 2012; 344:220-226.).

It has been traditionally considered that the skeleton is an inert organ that act as the storage of calcium and phosphorus, protecting the internal organs. Recent studies have found that bone is an important endocrine organ, which is regarded as paradigm of integrative physiology, not only act as a target organ, but also act as an important organ to modulate system functions (Karsenty G, Ferron M. The contribution of bone to whole-organism physiology. Nature. 2012;481:314-320.) (Karsenty G, Oury F. Biology without walls: the novel endocrinology of bone. Annu Rev Physiol. 2012; 74:87-105.), it can also regulate peripheral vascular by bone-vascular axis (Thompson B, Towler DA. Arterial calcification and bone physiology: role of the bone-vascular axis. Nature Reviews Endocrinology. 2012;8:529-543.) . Bone not only contains osteoblasts, osteoclasts and bone cells, but also rich in endothelial cells, macrophages, nerves and adipose tissue, and more enriched a large number of hematopoietic stem cells, bone marrow stromal stem cells. There are two kinds of hematopoietic stem cell niche, the osteoblast niche and endothelial niche, that cooperatively regulate haematopoiesis stem cell proliferation, mobilization and differentiation (Bianco P. Bone and the hematopoietic niche: a tale of two stem cells. Blood. 2011;117:5281-5288.) (Morrison SJ, Scadden DT. The bone marrow niche for haematopoietic stem cells. Nature. 2014;505:327-334.) .

In recent years, the pleiotropic effects of statins have attracted more and more attention. However, statins, as the rate limiting enzyme of cholesterol synthesis in the liver, less than 5% after oral administration reached the circulation and less than in the bone. We have found that intraosseous injection of statin could promote the secretion of insulin and enhance insulin sensitivity, and increased bone mass, improve bone mineral density and improve bone tissue micro structure, and enhance the mechanical properties of bone (CN201210596032.3, CN201210512630.8). US2014/323536 describes treatment of arterial wall by a combination of RAAS inhibitor and HMG-CoA reductase inhibitor. US2004/122077 describes HMG-CoA reductase inhibitors for promoting angiogenesis.

However, it has not previously been reported that intraosseous application of statins can mobilize endogenous endothelial progenitor cells and promote peripheral angiogenesis.

### Summary of the Invention

New progress in integrative physiology and the new discovery of "bone is an endocrine organ" may provide such a possibility. In particular, the present inventors are the first to appreciate the therapeutic potential of intervening at local bone to achieve the purpose of treatment of systemic diseases. The abundance of trabecular space and the bone marrow cavity provides an excellent space for intervention/local drug delivery.

Here, we found that a single local intraosseous application of a small dose of statins can continuously mobilize endogenous endothelial progenitor cells, significantly promote systemic angiogenesis, and treat the ischemic diseases.

Accordingly, one purpose of the present disclosure is to provide a composition comprising a statin or pharmaceutically acceptable salt thereof in the treatment of ischemic disease. Such compositions may be administered locally. According to the invention, such compositions are for intraosseous administration into the bone for the treatment of ischemic diseases.

According to the invention, intraosseous administration of statin or statin containing compositions locally into bone can achieve have promote angiogenesis systemically, and so can be used in the treatment of systemic ischemic diseases. Other conditions which would benefit from an increase in systemic angiogenesis may also be treated. In particular, as shown herein, therapeutic angiogenesis may be stimulated in organs such as the liver, spleen, kidney and pancreas. As a possibility, intraosseous administration of statin or statin containing compositions may be used to facilitate muscle growth, recover and/or repair, as well as skin and/or wound healing. Without wishing to be bound by theory, it is believed that the increase in angiogenesis enhances the formation of new muscle tissue (in the case of muscle growth, recover and/or repair), or new skin tissue to repair a wound. Other examples of conditions that may be treated include atherosclerosis. As demonstrated herein, the therapy can be used to effectively reduce plaque size and formation.

Specially, the present invention provides a new use of statin compounds for the treatment of ischemic diseases as a single dose for intraosseous administration.

In one specific embodiment of the invention, this invention provides a statin, or a composition comprising a statin or statin compounds for use in a method of treatment of ischemic disease, by intraosseous administration into the bone, wherein the ischemic disease is a peripheral ischemic disease, preferably diabetic acromelic ischemic disease.

In one specific embodiment of the invention, this invention provides a statin or a composition comprising a statin or statin compounds for use in a method of treatment of ischemic disease by intraosseous administration into the bone, wherein the ischemic disease is preferably a cardio-cerebrovascular ischemic disease.

In one specific embodiment of the invention, this invention provides a statin or a composition comprising a statin or statin compounds for use in a method of treatment of ischemic disease by intraosseous administration into the bone, wherein the composition comprises a statin compound or its pharmaceutically acceptable salt in a pharmacologically effective amount and pharmaceutically acceptable adjuvant material.

In one embodiment of the invention, the statin compound comprises, but not limited to, simvastatin, atorvastatin, fluvastatin, lovastatin, pravastatin, rosuvastatin, pitavastatin, bervastatin, cerivastatin, crilvastatin, dalvastatin, mevasatin or tenivastatin; preferably simvastatin. One or more statin, or a combination thereof, may be used.

The statin compound may be pharmaceutically acceptable salt, which comprises, but not limited to, hydrochloride, hydrobromide, hydriodate, sulfate, nitrate, phosphate, citrate, mesylate, trifluoroacetate, acetate, or salt of sodium, potassium, calcium, or magnesium thereof.

In one preferable embodiment of the invention, the statin compound is selected from simvastatin, atorvastatin, fluvastatin, lovastatin, pravastatin, rosuvastatin or pitavastatin, and the pharmaceutically acceptable salt thereof, for example hydrochloride, hydrobromide, sulfate, nitrate, phosphate, citrate, mesylate, or salt of sodium, potassium, calcium, or magnesium thereof; preferably simvastatin, atorvastatin calcium or atorvastatin sodium, fluvastatin sodium, pravastatin sodium, rosuvastatin calcium or pitavastatin calcium.

The present invention also relates to a pharmaceutical composition for intraosseous injection into bone for use in a method of treatment of ischemic disease, wherein said composition contains a statin compound or its pharmaceutically acceptable salt and a pharmaceutically acceptable carrier, diluent or excipient.

In one embodiment of the invention, the statin or composition comprising a statin or statin compound for intraosseous administration into the bone is preferably for administration via injection into bone or embedding into bone. The statin or composition for administration into the bone may be in the form of injectable dosage forms, wherein the injectable dosage forms include, but not limited to, the injectable solution, injectable suspension liquid, injectable emulsion, injectable gel, injectable solid form, or their slow or controlled release form, or their implant form. Here, the injectable solid form refers to those that is mixed with a solvent such as water for injection, normal saline injection or glucose solution for injection when it is used, to make it feasible for injection.

In one specific embodiment of the invention, the statin, or composition comprising a statin or statins compound for intraosseous administration into bone includes a statins compound or its pharmaceutically acceptable salt and a pharmaceutically acceptable adjuvant or adujvant material. Here, the pharmaceutically acceptable adjuvant or adjuvant material may be selected from at least one of the optional water-soluble solvent or oily solvent, dispersing agent, isotonic agent, preservative, solubilizer or stabilizers; water-soluble solvent can be selected from distilled water, normal saline, Ringer's solution or phosphate buffer (PBS); oil soluble solvent can be selected from vegetable oil, such as olive oil, castor oil, sesame oil, cottonseed oil or corn oil; dispersing agent can be selected from tween 20 or tween 80, polyethylene glycol, carboxy methyl cellulose, and/or sodium alginate; isotonic agent can be selected from chloride sodium, glycerol, sorbic alcohol, or glucose; Solubilizer can be selected from salicylic acid sodium, poloxamer or acetate sodium; preservative may be selected from methyl paraben, propyl paraben, benzyl alcohol, chlorobutanol, sodium benzoate, or phenol; stabilizer may be selected from albumin, such as human serum albumin, bovine serum albumin, etc. Moreover, said pharmaceutically acceptable adjuvant or adjuvant material may also be selected from biodegradable materials, such as polylactide, poly-1-lactide-glycolide, polyaspartic acid, and so on. For a person skilled in the art, the statin topical composition of the present invention can be prepared through known preparation technology. For example, the statin compound or its pharmaceutically acceptable salt together with dispersing agent, and/or isotonic agent, and/or preservative, and/or solubilizer and/or stabilizers are dissolved, suspended or emulsified in water-soluble solvent or oil-soluble solvent (Remington: the science and practice of pharmacy, 21st edition, 2005, Lippincott Williams).

Also described is a method of preparing a pharmaceutical composition, comprising a procedure of mixing a statin compound or its pharmaceutically acceptable salt in a therapeutically effective amount with a pharmaceutically acceptable carrier, diluent or excipient; for example, dissolving or suspending the statin compound or its pharmaceutically acceptable salt in a therapeutically effective amount in said pharmaceutically acceptable carrier, diluent or excipient.

In the embodiment of the invention, the dosing interval of the intraosseous administration of the statin medicament to mammalian is once every 7 days up to 600 days, preferably once every 10 to 500 days, more preferably once every 20 to 400 days, most preferably once every 30 to 300 days.

In terms of the intraosseous administration of the statin medicament to mammalian, a single dose of statin compound is from 0.1mg to 50mg, preferably from 0.5mg to 10mg. A clinician can adjust or modify the frequency and dose of administration according to needs of the clinical effect.

In an embodiment of the invention, a mammal is preferably human being.

The experimental data herein shows that the local single intraosseous administration of statins composition described herein significantly promote the mobilization of endogenous endothelial progenitor cells, contribute to the angiogenesis in the hind limb ischemia and skin defect model of diabetic rats, reduce atherosclerosis in ApoE^{-/-} mice. Considering the mobilization of EPCs is seriously damaged in diabetes and hyperlipidemia, it could be believed that local single intraosseous administration of statins or their composition could take more effective on angiogenesis to treat ischemia, which was verified in the middle cerebral artery occlusion (MCAo) induced cerebral ischemia model. It acts as therapeutic angiogenesis to treat ischemic diseases, improve microcirculation.

### Description of the Drawings

The present invention will be further explained with reference to the drawings.
Figure 1 shows that local single intraosseous injection of small dose of simvastatin promotes endogenous EPCs mobilization for a long period in type 1 diabetic rats.
Figure 2 shows that the local single intraosseous injection of small dose of simvastatin promotes endogenous EPCs mobilization after the hind limb ischemia in type 1 diabetic rats.
Figure 3 shows the effect of oral administration of high dose of simvastatin on endogenous EPCs mobilization after the hind limb ischemia in type 1 diabetic rats.
Figure 4 shows that the local single intraosseous injection of small dose of simvastatin improve microcirculation in type 1 diabetic rats.
Figure 5 shows that the local single intraosseous injection of small dose of simvastatin improve angiogenesis around pancreas site in type 1 diabetic rats.
Figure 6 show that the local single intraosseous injection of small dose of simvastatin improve systemic angiogenesis in type 1 diabetic rats.
Figure 7-1 shows that the local single intraosseous injection of small dose of simvastatin improve the angiogenesis after hind limb ischemia in type 1 diabetic rats.
Figure 7-2 shows that the local intraosseous injection of small dose of simvastatin accelerate the recovery of blood flow speed in hind limb ischemia of type 1 diabetic rats.
Figure 8-1 ultrasonic Doppler blood flowmeter shows that oral administration of simvastatin 20mg/kg/d for 3 weeks did not significantly restored the blood flow velocity in hind limb ischemia of type 1 diabetic rats.
Figure 8-2 shows that oral administration of high dose of simvastatin could not accelerate the recovery of blood flow speed in hind limb ischemia of type 1 diabetic rats.
Figure 9 reports the angiography results showing that the local intraosseous injection of small dose of simvastatin significantly increased angiogenesis (A). In contrast, no angiogenesis was observed in the group of oral administration of high dose of simvastatin (B).
Figure 10 shows that the local intraosseous injection of small dose of simvastatin promote the recovery of gastrocnemius atrophy induced by hind limb ischemia.
Figure 11 shows that the local intraosseous injection of small dose simvastatin promote the skin wound healing in STZ induced type 1 diabetic rats. A, B: the healing of skin wound, C, D: the capillary density.
Figure 12 shows the therapeutic effect of local single injection of small dose simvastatin on atherosclerosis in ApoE^{-/-} mice. A, B: the area of plaque en face, C: the area of plaque at aortic root, J: the number of EPCs in the circulation.
Figure 13 shows that the local single intraosseous injection of small dose of simvastatin promotes angiogenesis after middle cerebral artery occlusion in rats. Upper: angiograph of cerebral artery, lower: the TTC staining showing the ischemic area.

### Detailed Description of the Invention

The present invention is described in detail using examples given below.

### Preparation Examples

### Preparation of intraosseous injective simvastatin solution

Simvastatin (1000mg) was dissolved in 10 ml phosphate-buffered saline (PBS) containing 2% dimethylsulfoxide (DMSO; Sigma, USA) and 0.1 % bovine serum albumin (BSA, Sigma, USA), the obtained solution is homogeneous.

### Preparation of intraosseous injective thermosensitive simvastatin hydrogel

Poloxamer 407 (BASF, Ludwigshafen, Germany; 25 % w/w) was added to isotonic phosphate-buffered saline (PBS, pH 7.4, 4 °C) with gentle mixing until complete dissolution.

Gels loaded with simvastatin (National Institutes for Food and Drug Control, Beijing, China) were prepared by adding the drug to the prepared poloxamer 407 solutions. The final simvastatin concentrations were 0, 0.5, or 1 mg/ml.

### Example 1

### Long-term mobilization of endogenous EPCs in type 1 diabetic rats after single local intraosseous injection of small dose simvastatin

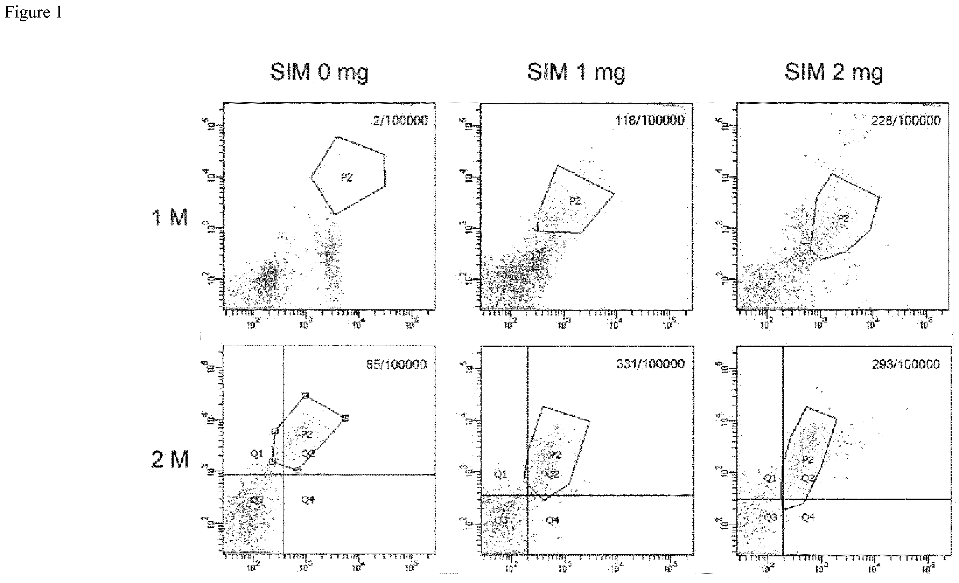

In the STZ induced type 1 diabetic rat model, we found that 1 month and 2 months after single local tibial intraosseous injection of simvastatin, the results showed that the circulated number of EPCs in the simvastatin group significantly increased, which were verified by FACS (see Figure 1).

### Example 2

### Comparison the effects of mobilization of endogenous EPCs between intraosseous injection of single small dose of simvastatin and oral administration of high dose simvastatin in the hind limb ischemia model of type 1 diabetic rats

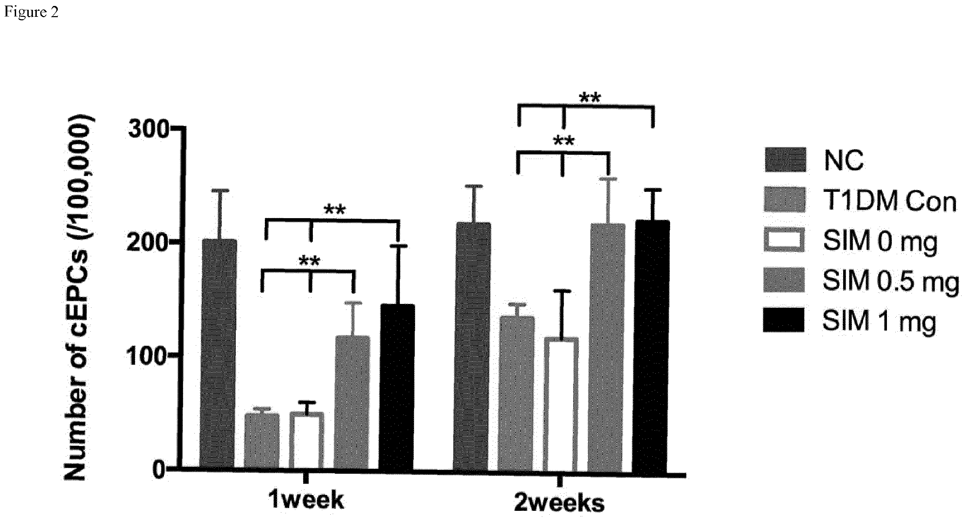

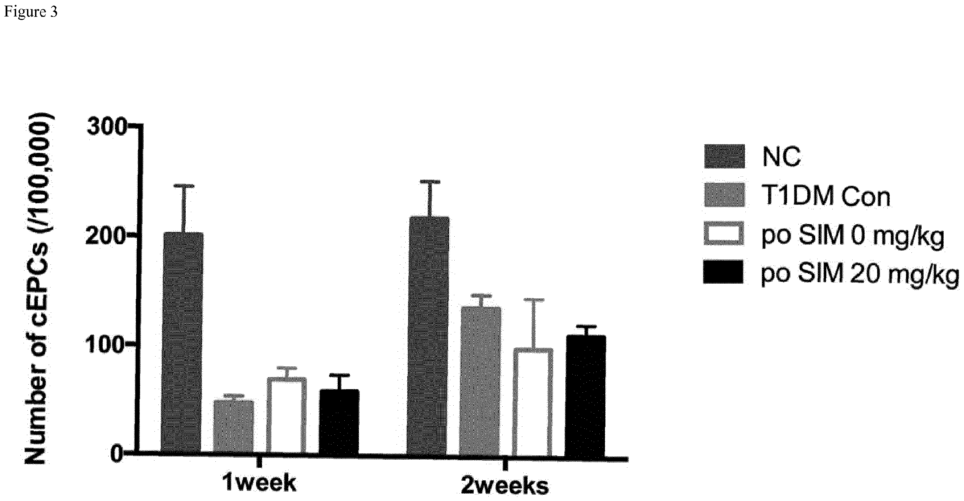

The right side of the rat femoral artery was removed by 1 cm segment in the STZ induced type 1 diabetic rats. The left tibia received local single intraosseous injection with 0g, 0.5mg, 1mg simvastatin respectively, the number of circulated EPCs were detected by FACS. The results showed that local single intraosseous injection of simvastatin can significantly increase the number of EPCs in the circulation (P<0.01) (see Figure 2). However, in the daily administration of 20mg/kg simvastatin group, the result of FACS showed that the oral administration of high dose simvastatin did not significantly increase the number of EPCs in the circulation (results see Figure 3). The results implied that different routes of administration of simvastatin might produce different effects.

### Example 3

### Local intraosseous injection of small dose simvastatin promotes systemic angiogenesis

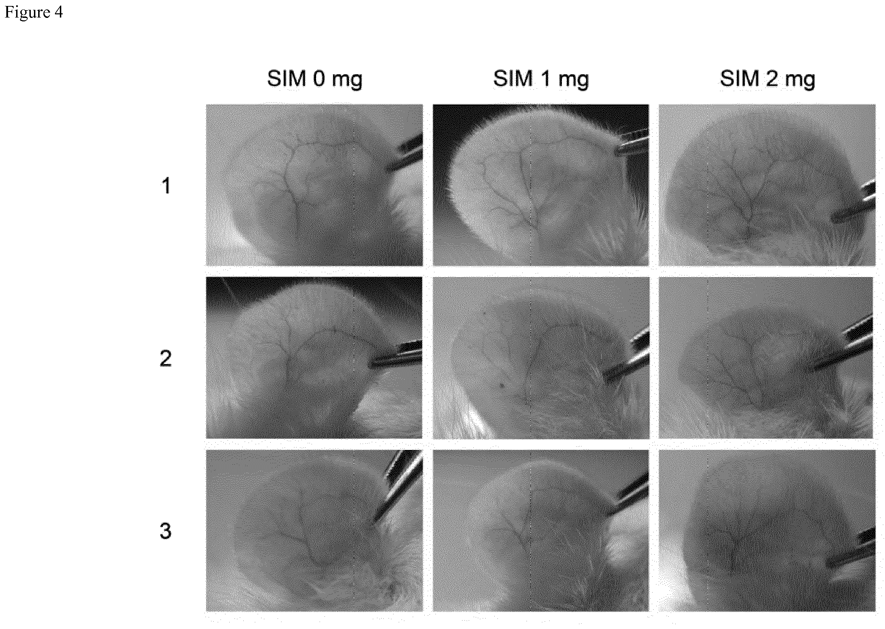

In the STZ induced type 1 diabetic rat, we found that local single intraosseous injection of small dose simvastatin (1mg or 2mg) promotes angiogenesis, the ear collateral circulation significantly increased 30 days later (see Figure 4).

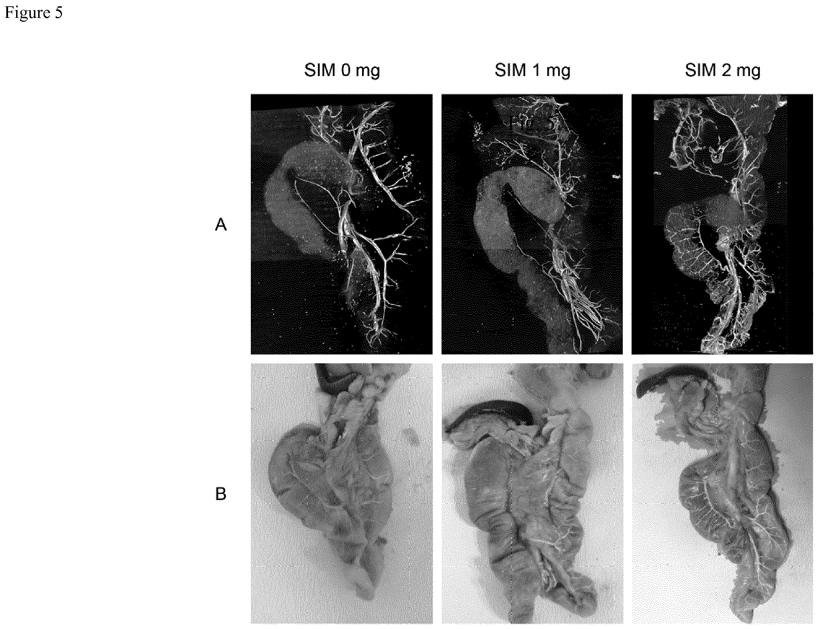

The rats were sacrificed before perfusion microfil®; and scanned by micro-CT. The angiography results showed that the local pancreatic vessels were significantly increased in the group of intraosseous injection of simvastatin, angiogenesis in the pancreas will protect islet cells (see Figure 5).

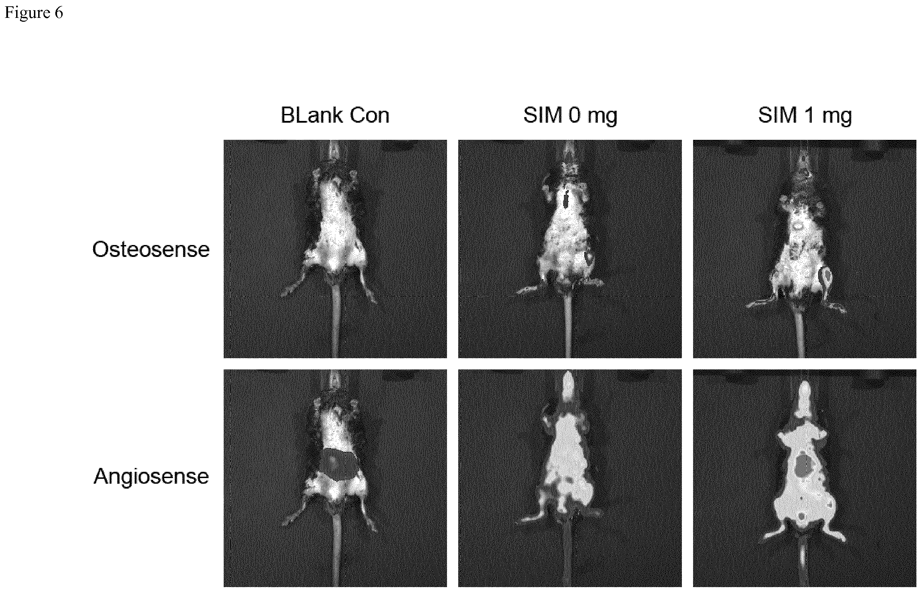

C57 mouse received local intraosseous injection of simvastatin 0mg or 1mg, four weeks after the tail vein injection of Osteosense® and Angiosense® fluorescent probes. The results showed that, 4 weeks after local intraosseous injection of simvastatin, the injection site was still active on bone formation, and the liver, spleen, kidney and pancreas were detected with strong vascular signal (see Figure 6).

### Example 4

### Local intraosseous injection of small dose simvastatin promotes angiogenesis of the contra-lateral limb ischemia of STZ induced type 1 diabetic rats

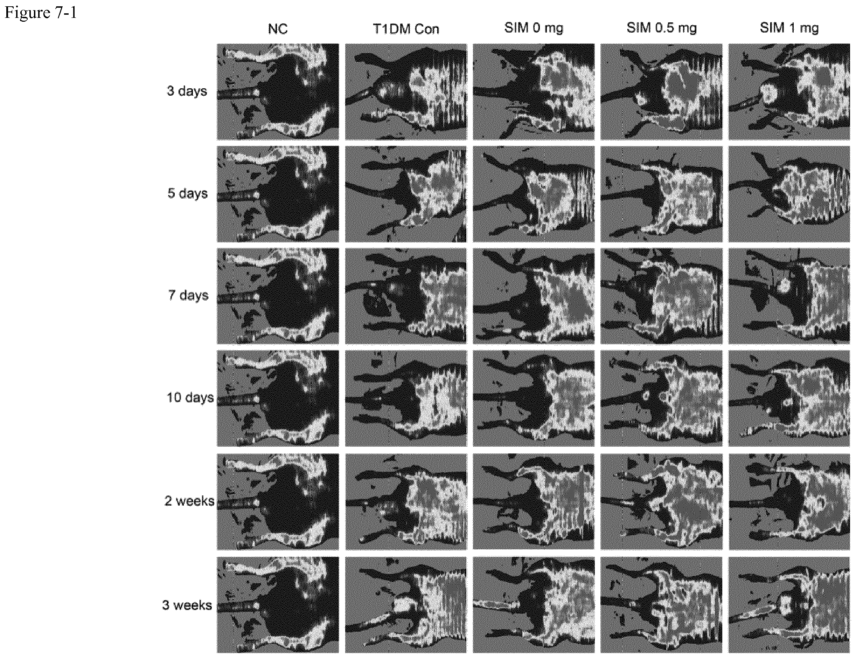

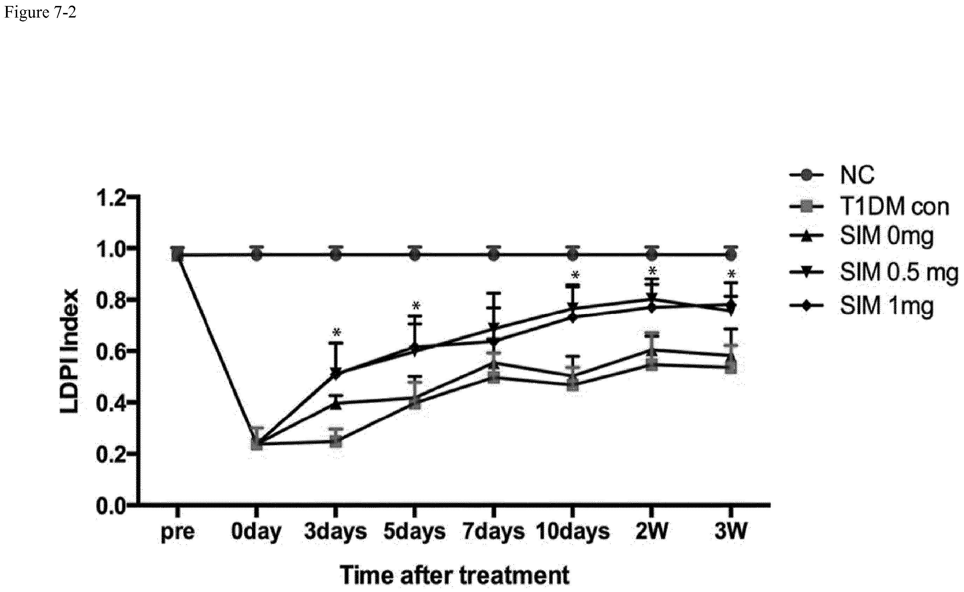

In the STZ induced type 1 diabetic rats, the right side of the rat femoral artery was removed by 1.0cm segment. The rat left tibia were received single local intraosseous injection of 0g, 0.5mg, 1 mg of simvastatin respectively, the ultrasonic Doppler blood flowmeter detected that local intraosseous injection of small dose of simvastatin accelerate the recovery of blood flow speed (P< 0.01), the earliest in the 3 days can be observed in some blood recovery (results see Figure 7-1, 2).

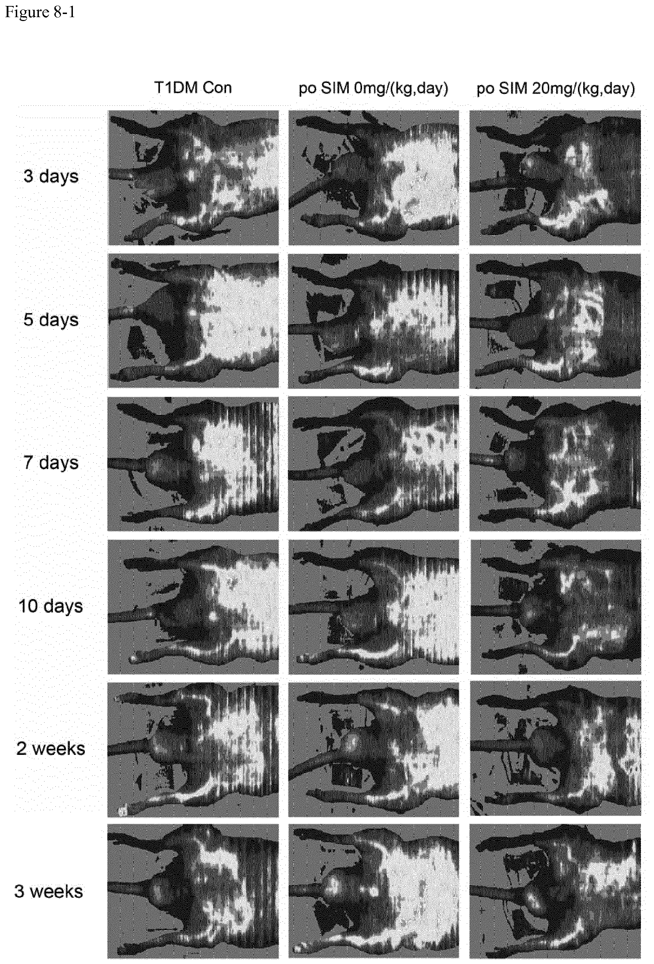

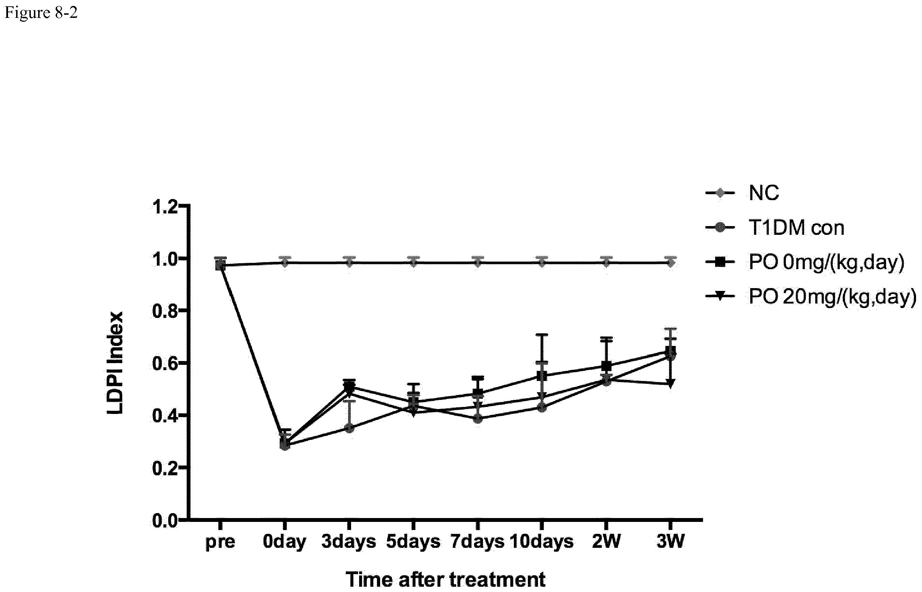

The STZ induced type 1 diabetic rat model of hind limb ischemia received daily gavage of 20mg/kg/d simvastatin for 3 weeks, the results of the ultrasonic Doppler blood flowmeter showed that oral administration of simvastatin 20mg/kg/d for 3 weeks did not significantly restored the blood flow velocity (see Figure 8-1, 2).

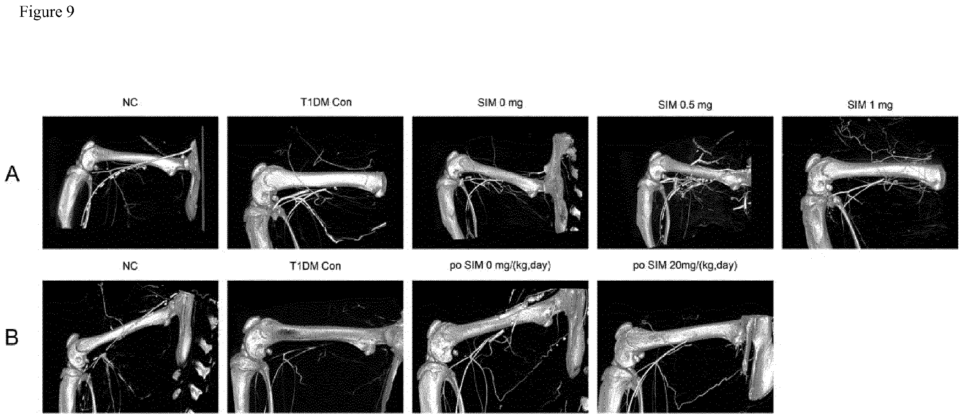

The rats were sacrificed before perfusion microfil®; and scanned by micro-CT to observe the local pancreatic vessels. The angiography results showed that the local intraosseous injection of small dose of simvastatin significantly increased angiogenesis, there was more collateral circulation than the control group. While, there were no angiogenesis observed in the group of oral administration of high dose of simvastatin (see Figure 9).

### Example 5 (not part of the claimed invention)

### Local intraosseous injection of small dose simvastatin promotes recovery the ratio of wet weight of gastrocnemius

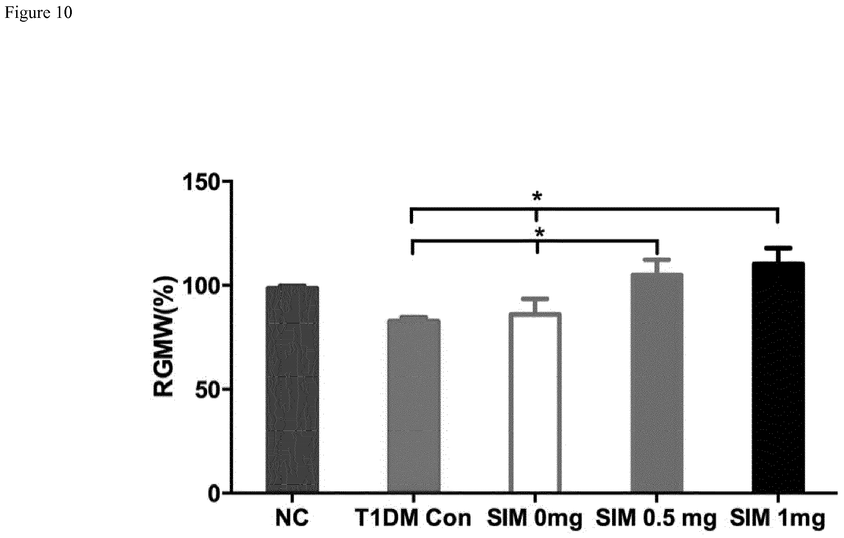

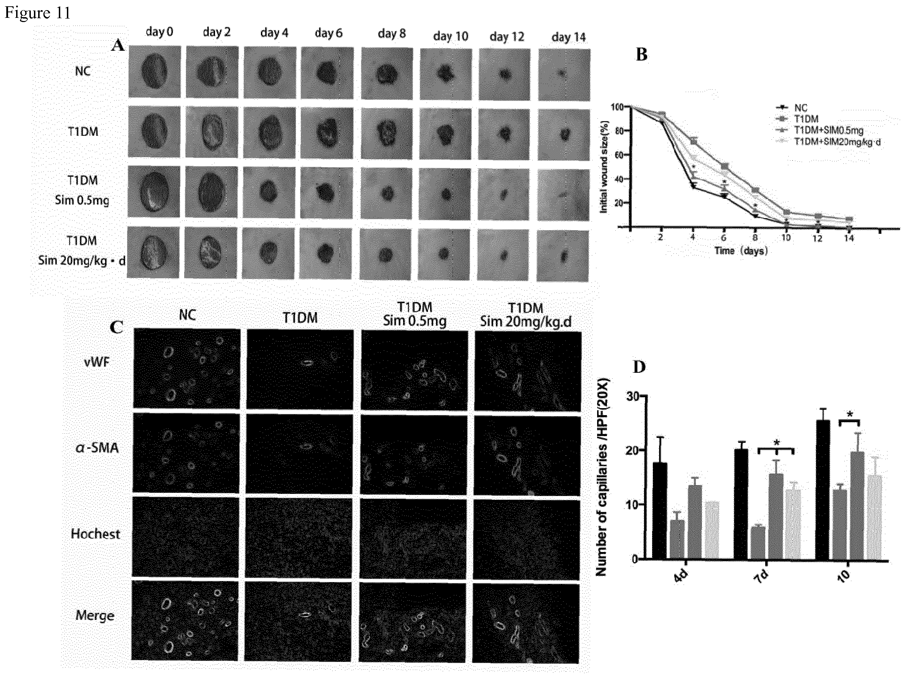

In the STZ induced type 1 diabetic rats, the right side of the rat femoral artery was removed by 1.0cm segment. The rat left tibia were received single local intraosseous injection of 0g, 0.5mg, 1 mg of simvastatin respectively, and the gastrocnemius muscle was measured after 4 weeks. The results showed that the ischemic gastrocnemius wet weight significantly atrophied in the simvastatin 0mg group (P<0.05), while, there were no obvious atrophy in the group of local intraosseous injection of simvastatin 0.5mg (see Figure 10).

### Example 6 (not part of the claimed invention)

### Effect of local intraosseous injection of small dose simvastatin on skin wound healing in STZ induced type 1 diabetic rats

In the STZ induced Type 1 diabetic rats, using the skin sampling device to cause a skin defect (ϕ=12mm) on the back of the rats. The left tibia of rats received local intraosseous injection of 0g, 0.5mg simvastatin respectively. The results showed that the local intraosseous of simvastatin can significantly accelerate the skin defect recovery rate (P<0.01), and the effect is superior to that of oral high dose simvastatin (20mg/kg/d). Immunofluorescence staining of new blood vessels also showed that simvastatin could significantly increase the angiogenesis (see Figure 11).

### Example 7 (not part of the claimed invention)

### Therapeutic effect of local single intraosseous injection of small dose simvastatin on atherosclerosis in ApoE-/- mice

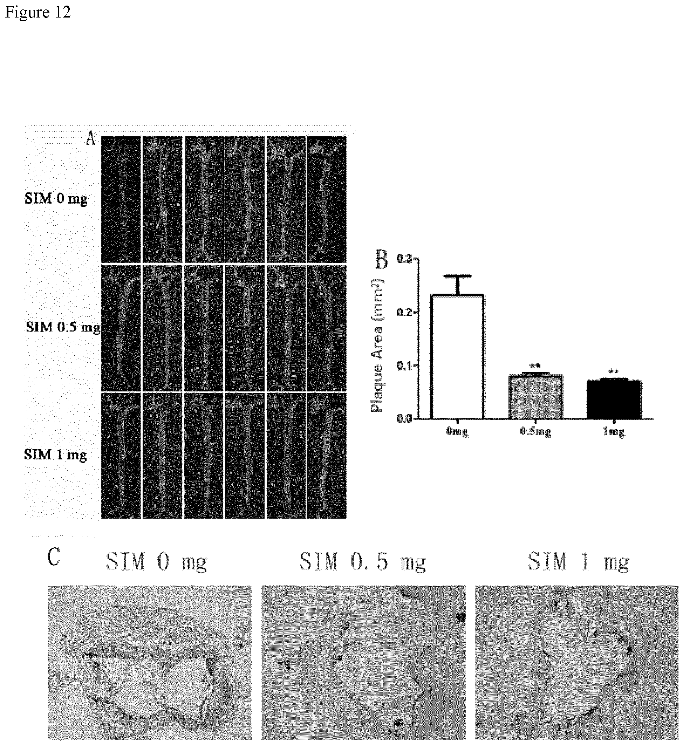

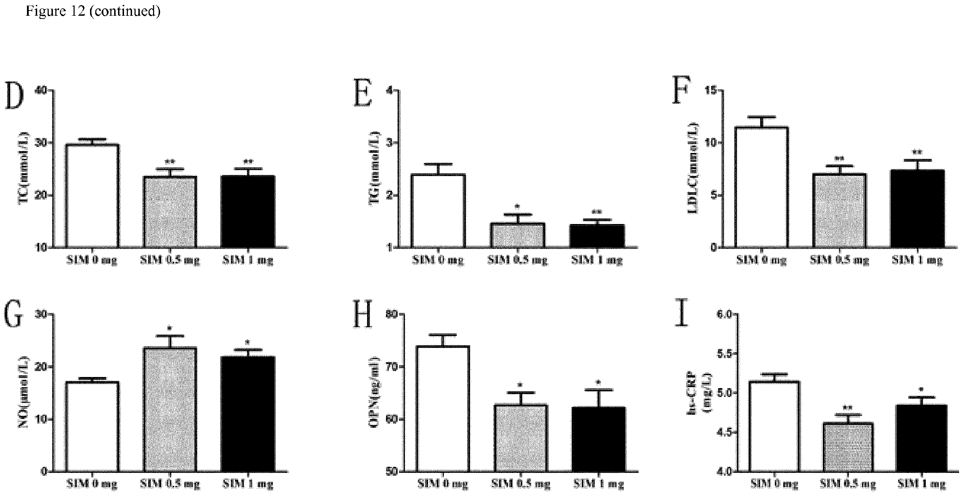

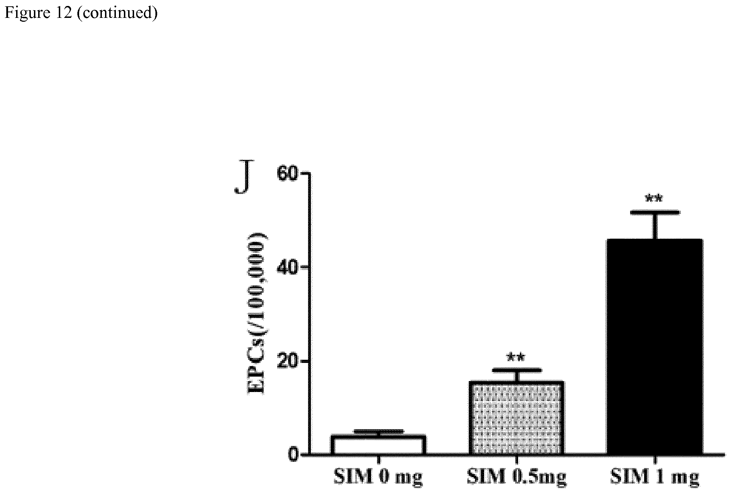

The high fat diet induced atherosclerosis ApoE-/- mice received local single intraosseous injection of simvastatin (0mg, 0.5mg, 1mg). The animals were euthanized 8 weeks later. The *en face* of aorta and aortic root continuous sections and stained by oil red O staining and image analysis software is calculated by comparing the size of plaque, blood lipid concentrations were detected, the serum nitric oxide, osteopontin and hypersensitive C reaction protein was detected by ELISA, the number of peripheral blood EPCs were detected by FACS. The results showed that in the local intraosseous injection of small dose simvastatin group, the plaque was significantly reduced, total cholesterol, triglyceride and low density lipoprotein cholesterol significantly decreased, osteopontin and high-sensitivity C-reactive protein concentration were significantly decreased; while the concentration of serum nitric oxide significantly increase. Most importantly, the number of EPCs in peripheral blood significantly increased (see Figure 12).

### Example 8

### Local single Intraosseous injection of small dose of simvastatin promote angiogenesis after middle cerebral artery occlusion in rats

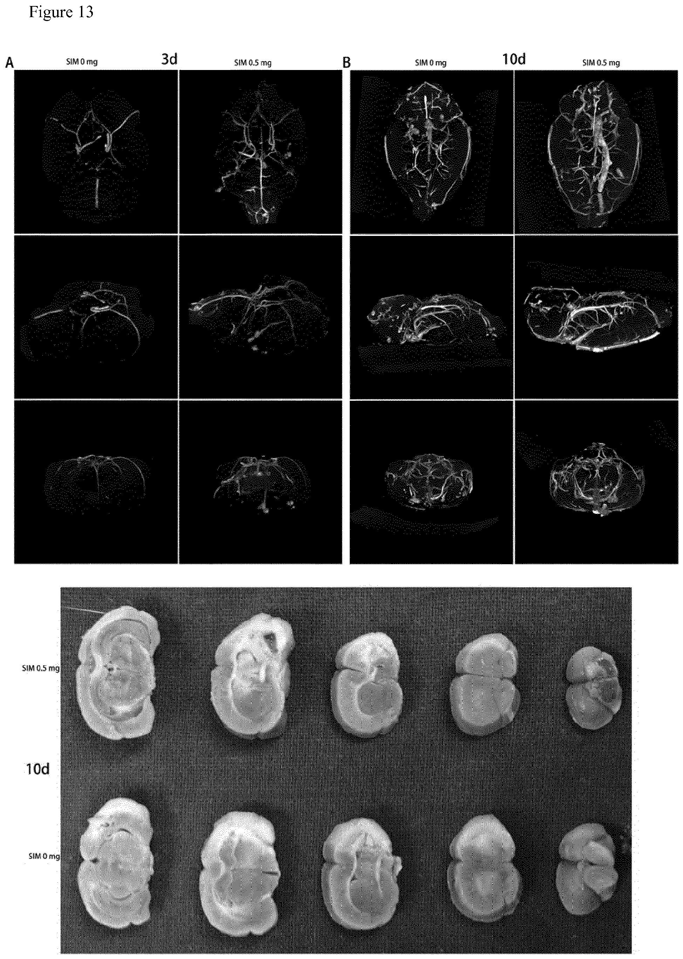

In rat middle cerebral artery occlusion (MCAo) induced cerebral ischemia animal models, the rats received a local single intraosseous injection of simvastatin 0.5mg. Three days later, the microfill® perfusion and micro-CT scanning showed that in the experimental group, the collateral circulation reconstruction was significantly higher than that of the control group. TTC staining display infarct area was significantly lower than that in control group (see Figure 13).

## Claims

1. A composition comprising a statin or a pharmaceutically acceptable salt thereof for use in a method of treatment of an ischemic disease, disorder or condition in a mammal, wherein said composition is for administration as a single dose by intraosseous application into bone.

2. The composition for use according to claim 1, wherein the statin is selected from simvastatin, atorvastatin, fluvastatin, lovastatin, pravastatin, rosuvastatin, pitavastatin, bervastatin, cerivastatin, crilvastatin, dalvastatin, mevasatin and tenivastatin.

3. The composition for use according to claim 1 or 2, wherein the statin is simvastatin.

4. The composition for use according to any one of the preceding claims, wherein said composition further comprises a pharmaceutically acceptable adjuvant.

5. The composition for use according to any one of the preceding claims, wherein said pharmaceutically acceptable salt is selected from hydrochloride, hydrobromide, hydriodate, sulfate, nitrate, phosphate, citrate, mesylate, trifluoroacetate, and acetate, or salt of sodium, potassium, calcium, or magnesium thereof.

6. The composition for use according to any one of the preceding claims, wherein said mammal is a human being.

7. The composition for use according to any one of the preceding claims, wherein said ischemic disease is a peripheral ischemic disease.

8. The composition for use according to claim 7, wherein said peripheral ischemic disease is diabetic acromelic ischemic disease.

9. The composition for use according to any one of claims 1 to 6, wherein said ischemic disease is a cardio-cerebrovascular ischemic disease.

10. The composition for use according to any one of the preceding claims, wherein the composition is for administration at a dosing interval of once every 7 days up to 600 days, preferably once every 10 to 500 days, more preferably once every 20 to 400 days, most preferably once every 30 to 300 days.

11. The composition for use according to any one of the preceding claims, wherein the single dose of the statin or pharmaceutically acceptable salt thereof is from 0.1mg to 50mg, preferably from 0.5mg to 10mg.

## Patentansprüche

1. Zusammensetzung, umfassend ein Statin oder ein pharmazeutisch verträgliches Salz davon zur Verwendung in einem Verfahren zur Behandlung einer ischämischen Erkrankung, Störung oder eines ischämischen Zustands bei einem Säuger, wobei die genannte Zusammensetzung zur Verabreichung als eine Einzeldosis mittels intraossärer Applikation in Knochen vorgesehen ist.

2. Zusammensetzung zur Verwendung nach Anspruch 1, wobei das Statin ausgewählt ist aus: Simvastatin, Atorvastatin, Fluvastatin, Lovastatin, Pravastatin, Rosuvastatin, Pitavastatin, Bervastatin, Cerivastatin, Crilvastatin, Dalvastatin, Mevastatin und Tenivastatin.

3. Zusammensetzung zur Verwendung nach Anspruch 1 oder 2, wobei das Statin Simvastatin ist.

4. Zusammensetzung zur Verwendung nach einem der vorangehenden Ansprüche, wobei die genannte Zusammensetzung ferner ein pharmazeutisch verträgliches Adjuvans umfasst.

5. Zusammensetzung zur Verwendung nach einem der vorangehenden Ansprüche, wobei das genannte pharmazeutisch verträgliche Salz ausgewählt ist aus: Hydrochlorid, Hydrobromid, Hydroiodat, Sulfat, Nitrat, Phosphat, Citrat, Mesylat, Trifluoracetat und Acetat, oder einem Natrium-, Kalium-, Calcium- oder Magnesiumsalz.

6. Zusammensetzung zur Verwendung nach einem der vorangehenden Ansprüche, wobei der genannte Säuger ein Mensch ist.

7. Zusammensetzung zur Verwendung nach einem der vorangehenden Ansprüche, wobei die genannte ischämische Erkrankung eine periphere ischämische Erkrankung ist.

8. Zusammensetzung zur Verwendung nach Anspruch 7, wobei die periphere ischämische Erkrankung eine akromelische ischämische Erkrankung bei Diabetes ist.

9. Zusammensetzung zur Verwendung nach einem der Ansprüche 1 bis 6, wobei die genannte ischämische Erkrankung eine kardiozerebrovaskuläre ischämische Erkrankung ist.

10. Zusammensetzung zur Verwendung nach einem der vorangehenden Ansprüche, wobei die Zusammensetzung zur Verabreichung bei einem Dosierungsintervall von einmal alle 7 Tage bis zu 600 Tagen, bevorzugt einmal alle 10 bis 500 Tage, bevorzugter einmal alle 20 bis 400 Tage, am bevorzugtesten einmal alle 30 bis 300 Tage vorgesehen ist.

11. Zusammensetzung zur Verwendung nach einem der vorangehenden Ansprüche, wobei die Einzeldosis des Statins oder des pharmazeutisch verträglichen Salzes davon von 0,1 mg bis 50 mg, bevorzugt von 0,5 mg bis 10 mg beträgt.

## Revendications

1. Composition comprenant une statine ou un sel pharmaceutiquement acceptable de celle-ci pour utilisation dans un procédé de traitement d'une maladie, d'un trouble ou d'un état ischémique chez un mammifère, où ladite composition est destinée à être administrée en une seule dose par application intraosseuse dans l'os.

2. Composition pour utilisation selon la revendication 1, où la statine est choisie parmi la simvastatine, l'atorvastatine, la fluvastatine, la lovastatine, la pravastatine, la rosuvastatine, la pitavastatine, la bervastatine, la cérivastatine, la crilvastatine, la dalvastatine, la mévastatine et la ténivastatine.

3. Composition pour utilisation selon la revendication 1 ou 2, où la statine est la simvastatine.

4. Composition pour utilisation selon l'une quelconque des revendications précédentes, où ladite composition comprend en outre un adjuvant pharmaceutiquement acceptable.

5. Composition pour utilisation selon l'une quelconque des revendications précédentes, où ledit sel pharmaceutiquement acceptable est choisi parmi le chlorhydrate, le bromhydrate, l'iodhydrate, le sulfate, le nitrate, le phosphate, le citrate, le mésylate, le trifluoroacétate et l'acétate, ou un sel de sodium, de potassium, de calcium ou de magnésium.

6. Composition pour utilisation selon l'une quelconque des revendications précédentes, où ledit mammifère est un être humain.

7. Composition pour utilisation selon l'une quelconque des revendications précédentes, où ladite maladie ischémique est une maladie ischémique périphérique.

8. Composition pour utilisation selon la revendication 7, où ladite maladie ischémique périphérique est une maladie ischémique acromélique diabétique.

9. Composition pour utilisation selon l'une quelconque des revendications 1 à 6, où ladite maladie ischémique est une maladie ischémique cardiocérébrovasculaire.

10. Composition pour utilisation selon l'une quelconque des revendications précédentes, où la composition est destinée à être administrée à un intervalle posologique d'une fois tous les 7 jours jusqu'à 600 jours, de préférence une fois tous les 10 à 500 jours, plus préférablement une fois tous les 20 à 400 jours, le plus préférablement une fois tous les 30 à 300 jours.

11. Composition pour utilisation selon l'une quelconque des revendications précédentes, où la dose unique de la statine ou du sel pharmaceutiquement acceptable de celle-ci va de 0,1 mg à 50 mg, de préférence de 0,5 mg à 10 mg.
